(19) Europäisches Patentamt
European Patent Office
Office européen des brevets

(11) Veröffentlichungsnummer: **0 314 828 B1**

(12) # EUROPÄISCHE PATENTSCHRIFT

(45) Veröffentlichungstag der Patentschrift: **29.05.91**

(51) Int. Cl.5: **A61K 31/47**

(21) Anmeldenummer: **87116188.1**

(22) Anmeldetag: **03.11.87**

Die Akte enthält technische Angaben, die nach dem Eingang der Anmeldung eingereicht wurden und die nicht in dieser Patentschrift enthalten sind.

(54) **4-Phenyl-1,2,3,4-tetrahydroisochinoline als Ulcusmittel.**

(43) Veröffentlichungstag der Anmeldung:
**10.05.89 Patentblatt 89/19**

(45) Bekanntmachung des Hinweises auf die Patenterteilung:
**29.05.91 Patentblatt 91/22**

(84) Benannte Vertragsstaaten:
**BE CH DE ES FR GB LI NL SE**

(56) Entgegenhaltungen:
**EP-A- 0 161 501**
**FR-A- 2 528 044**

**ARZNEIMITTELFORSCHUNG, Band 30(I), Nr. 1, 1980, Seiten 69-73; M. BICKEL: "Antiulcer effects of nomifensine, a new antidepressant, on stress-induced ulcers in the rat"**

**CHEMICAL ABSTRACTS, Band 95, Nr. 19, 9. November 1981, Seite 724, Zusammenfassung Nr. 169011b, Columbus, Ohio, US; & ES-A-490 806 (LABORATORIOS PREM S.A.) 16-02-1981**

(73) Patentinhaber: **S O "PHARMACHIM"**
**Iliensko Chaussée 16**
**Sofia(BG)**

(72) Erfinder: **Borissov Ivanov, Chavdar**
**Komplex Mladost-1, Bl. 46-4**
**Sofia(BG)**
Erfinder: **Stoyanova Ivanova, Nedyalka**
**11, T. Kostov Street**
**Sofia(BG)**
Erfinder: **Dimitrov Paskov, Vladimir**
**36, Kiril i Metodi Street**
**Sofia(BG)**
Erfinder: **Dimitrova Daleva, Lilyana**
**Bl. 20, Persenk Street**
**Sofia(BG)**
Erfinder: **Minkova Mondeshka, Diana**
**125, Rakovski Street**
**Sofia(BG)**
Erfinder: **Dimitrova Berova, Nikolina**
**Komplex Kr. Selo, Bl. 194-B**
**Sofia(BG)**
Erfinder: **Stefanova Rakovska, Rossitza**
**Bl. 37, Boul. Vapzarov**
**Sofia(BG)**

**EP 0 314 828 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JPN. J. PHARMACOL., Band 33, Zusatz, 1983, Seite 219P; C. HARA et al.: "Effect of various antidepressants on development of activity-stress ulcer in rats"

J. CLIN. PSYCHIATRY, Band 46, Nr. 10II, 1985, Seiten 6-22; W.K. GOODMAN et al.: "Therapeutic applications and mechanisms of action of monoamine oxidase inhibitor and heterocyclic antidepressant drugs"

MOD. MED., Band 6, Nr. 20, 1978, Seiten 1360-1369; L. BLAHA et al.: "Psychopharmaka bei der Behandlung der Ulkuskrankheit?"

INT. PHARMACOPSYCHIAT., Band 17, Zusatz 1, 1982, Seiten 4-20; J. HOFFMANN: "Pharmacology of nomifensine"

Erfinder: Nikolova Tosheva, Donka

Kavarna Town(BG)
Erfinder: Yantshev Zaykov, Hristo
Komplex Lyulin Boul. Z. Stoyanov, Bl. 401
Sofia(BG)
Erfinder: Nissim Nissimov, Yossif
37, Sofronii Street
Sofia(BG)
Erfinder: Lyubomirov Orachev, Vesselin
17, Dede-Agach Street
Sofia(BG)
Erfinder: Nikolova Nacheva, Rumyana
25, Boul. Dondukov
Sofia(BG)
Erfinder: Nestorova Tancheva, Chonka
3, Miyazi Street
Sofia(BG)
Erfinder: Georgieva Angelova, Ivanka
19, G. Genov Street
Sofia(BG)
Erfinder: Emilov Boyadjiev, Stefan
4b, Sheynovo Street
Sofia(BG)
Erfinder: Minchev Mechkov, Grigor
Bl. 9, Boul. St. Lepoev
Sofia(BG)
Erfinder: Krumov Dimitrov, Boris
39, Boul. 9ti Septemvri
Sofia(BG)
Erfinder: Kcnstantinov Matov, Vladimir
45, Ivan Assen II Street
Sofia(BG)
Erfinder: Dimitrov Gendjev, Zdravko
Komplex Mladost-1, Bl. 27
Sofia(BG)

(74) Vertreter: von Füner, Alexander, Dr. et al
Patentanwälte v. Füner, Ebbinghaus, Finck
Mariahilfplatz 2 & 3
W-8000 München 90(DE)

## Beschreibung

Die Erfindung betrifft ein Ulkusmittel, insbesondere ein Ulkusmittel zur Behandlung von Streß-, akuten und chronischen Magen- und Zwölffingerdarm-Geschwüren und zur Prophylaxe bei allen Zuständen, die zur Bildung von Geschwüren führen.

Es ist bekannt, daß das sauere Magensekret durch Histamin-$H_2$-Rezeptoren stimuliert wird. Es ist ebenfalls bekannt, daß die Antagonisten der $H_2$-Rezeptoren das sauere Magensekret bei Mensch und Tier inhibieren und eine praktische Anwendung zur Behandlung der Geschwüre finden. Solche Antagonisten der $H_2$-Rezeptoren sind: N-Cyan-N'-methyl-N"-2-[[(5-methyl-1H-imidazol-4-yl)methyl]thio]-ethylguanidin, Cimeditin (US-Patent 3950353) und N-[2-[[[-5-[(Dimethylamino)-methyl])-2-furanyl]methyl]-thio]ethyl]-N'-methyl-2-nitro 1,1-ethan-diamin "Ranitidin" (GB-Patent 1565966).

Diese Antagonisten sind wirksam bei der Unterdrückung des vom Histamin und Pentagastrin stimulierten saueren

Magensekrets sind aber weniger aktiv bei der Behandlung von Streßgeschwüren. Außerdem besitzen sie einige unerwünschte Nebeneffekte bei ihrer systematischen Anwendung. So kann z.B. das Cimetidin eine Erosion der Magen-Zwölf-fingerdarm mukösen Membran, Magenkrebs, Bradykardie, Leukopenie, Agranulocytose und hämolytische Hepatitis hervorrufen.

Andererseits ist es bekannt, daß das 2-Methyl-4-phenyl-8-amino-1,2,3,4-tetrahydroisochinolin-saueres-Maleat unter der Handelsbezeichnung "Alival", bisher in der medizinischen Praxis als Antidepressant angewandt, bei Experimentalmodellen von Streßgeschwüren bei Mäusen, einen Antigeschwüreffekt aufweist, aber zum Unterschied von den Histamin-$H_2$-Antagonisten das sauere Magensekret nicht inhibiert. Die Antigeschwüraktivität dieser Verbindung ist kleiner als die der bekannten Antigeschwürpräparate (M. Bickel, Arzn.Forsch. 30 (1), 69, 1980). In letzter Zeit wurden unterwünschte klinische Nebeneffekte des oben erwähnten Präparats festgestellt, was dessen Anwendung bei der Behandlung von Geschwüren problematisch macht.

In der FR-A-2 528 044 werden Verbindungen der Formel

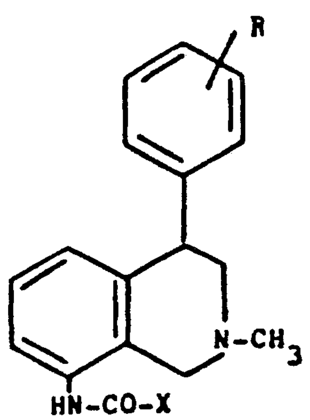

beschrieben. Diese Verbindungen sollen einen antidepressiven Effekt besitzen.

In der EP-A-0 161 501 werden Antiulcermittel auf der Basis von Histamin $H_2$ Blocker beschrieben, die zusätzlich ein Antidepressant der allgemeinen Formel

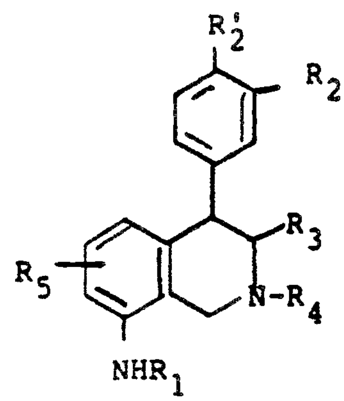

enthalten.

In dem Artikel von L. Blaha, Mod. Med., Bd. 6,. Nr. 20, 1978, S. 1360-1369 (insbesondere Tabelle 2, Alival) werden Antidepressiva mit Dosierungsvorschlägen aufgeführt, wobei ausgeführt wird, daß neben den modernen, mit weniger Nebenwirkungen belasteten Substanzen - z.B. Ludiomil®, Mianserin®, Alival®, - die Behandlung mit den "klassischen" tricyclischen Antidepressiva Vorteile bringen kann.

In Jpn. J. pharmacol., Vol. 33, Suppl, 1983, S. 219P, wird "the effect of various antidepressants on development of activity-stress ulcer in rats" beschrieben. Dabei wird auch das Nomifensin erwähnt.

Aufgabe der Erfindung ist es, ein Ulkusmittel mit hoher Aktivität bezüglich Magen- und Zwölffingerdarmgeschwüren und besonders von Streßgeschwüren, mit minimaler Toxizität und schwach ausgeprägten Nebeneffekten bei seiner systematischen Anwendung, bereitzustellen.

Erfindungsgemäß wird die Verwendung von 2-Methyl-4-(4-chlorphenyl)-8-ethoxycarbonyl amino-1,2,3,4-tetrahydroisoquinolin (±)I, seines rechtsdrehenden Isomers (+)I und/oder seiner optisch aktiven Isomeren zur Herstellung von Ulkusmitteln vorgeschlagen.

Als Ergebnis von genauen pharmakologischen Untersuchungen an Experimentalmodellen von Streß-, Indometazin-, Reserpingeschwüren und Geschwüren nach Shei wurde festgestellt, daß die erfindungsgemäß vorgeschlagenen Verbindungen eine unerwartet hohe Antigeschwüraktivität besitzen, die bei den angegebenen Verbindungen: 2-Methyl-4-(4-chlorphenyl)-8-ethoxycarbonylamino-1,2,3,4-tetrahydroisochinolin (±)I und seinen optisch aktiven Isomeren, insbesondere rechtsdrehenden Isomer (+)I besonders ausgeprägt ist.

Diese Verbindungen können für die Behandlung von Streß-, akuten und chronischen Zwölffingerdarm- und Magengeschwüren in Form von Tabletten, Kapseln oder Dragees, in denen der aktive Wirkstoff in Verbindung mit trivialen pharmazeutischen Hilfsstoffen enthalten sein muß, eingesetzt werden.

Die fertigen Arzneiformen sind unter Anwendung von bekannten technologischen Methoden zu erhalten.

Ein Ulkusmittel mit dem Wirkstoff besitzt erfindungsgemäß einen mehrfach stärker ausgeprägten Ulkuseffekt bei Streßgeschwüren, durch Indometazin und Reserpin hervorgerufenen Geschwüren und bei Geschwüren nach Shei, im Vergleich zu den bekannten Histamin-$H_2$-Antagonisten.

Außerdem wird der Ulkuseffekt schon bei Anwendung von sehr kleinen Dosen erzielt.

Die erfindungsgemäß verwendbaren Verbindungen besitzen eine niedrige Toxizität, was auch die minimalen Nebenwirkungen bei Ihrer Anwendung erklärt.

Das racemische 2-Methyl-4-(4-chlorphenyl)-8-ethoxycarbonylamino-1,2,3,4-tetrahydroisochinolin (±)I ist als Antidepressions mittel in DE 3 320 028 beschrieben.

Die Verbindungen (±)I wurden nach der in der DE 3 320 028, E.Z. Kaczian, L. Gyorgy und G. Deak I. Med. Chem. 29, 1189-95, 1986 beschriebenen Methode hergestellt, wobei ihre physikalisch-chemischen Eigenschaften mit den in der Literatur angegebenen übereinstimmen.

Die bis jetzt in der Literatur nicht beschriebenen optisch aktiven Isomere wurden mit Hilfe eines zweistufigen Verfahrens erhalten. Es besteht darin, daß

a) eine optische Teilung des racemischen 2-Methyl-4-(4-chlorphenyl)-8-amino-1,2,3,4-tetrahydroisochinolins (±)II erfolgt und

b) die optisch aktiven Isomere (-)II und (+)II mit Ethylchlorformiat behandelt werden.

Der racemische II wurde nach der im J. Med. Chem. 29, 1189-95, 1986 beschriebenen Methode erhalten.

Das optisch aktive (-)2-Methyl-4-(4-chlorphenyl)-8-amino-1,2,3,4-tetrahydroisochinolin (-)II erhält man aus dem racemischen II bei Anwendung eines Trennagenten /-/-/ 2R, 3R(-0,0-Di-)p-toluyl-Weinsäure in Isopropanollösung. Das durch zweifaches Umkristallisieren aus Isopropanol isolierte diastereomere Salz

wird in eine freie Base durch Behandlung mit einer Ammoniak-Wasserlösung umgewandelt. Das sich rechtsdrehende diastereoisomere Salz, das sich in der Mutterlauge der Trennung befindet, wird in eine freie Base umgewandelt, aus welcher durch Behandlung mit nicht naturalem /+/-/ 2 S, 3 S (-0,0-Di-)p-toluyl-Weinsäure das reine rechtsdrehende Isomer (+)II in Form einer freien Base erhalten wird.

Die optisch aktiven Ethoxycarbonylamino-Derivate (+) und (-) I erhält man aus den optisch aktiven (-)II und (+)II mittels Behandlung mit Ethylchlorformiat in Benzollösung unter Sieden.

Die folgenden Beispiele veranschaulichen die Erfindung:

## Beispiel 1

Bestimmung des Ulkuseffektes der erfindungsgemäß verwendeten Verbindungen anhand eines Experimentalmodells von Wasser-Immersions-Streßgeschwüren.

Die Versuche werden mit 280 weißen männlichen Ratten - Wistar-Rasse - mit einem Gewicht von 150 bis 200 g nach der Takagi K., Ishii Y., Arzneim. Forsch. Drug. Res. 17, 1544 (1967)-Methode durchgeführt und entsprechend modifiziert. Achtzehn Stunden vor dem Versuch werden die Tiere nicht gefüttert bei freiem Zugang zu Wasserquellen. Diese Verbindungen werden oral verabreicht; anschließend werden die Ratten, zur Verhinderung jeglicher Bewegung, auf dem Rücken an einer Unterlage angebunden, untereinander isoliert und in bis auf 17 - 18 °C temperiertes Wasser getaucht. Fünf Stunden später wurden die Tiere getötet, ihre Mägen herausgenommen und makroskopische destruktive Änderungen festgestellt.

Die Ergebnisse der pharmakologischen Untersuchungen sind anhand des experimentalen Modells von Wasser-Immersions-Streßgeschwüren in der Tabelle 1 angegeben.

Tabelle 1

Einfluß von (±)I, (+)I, (-)I auf ein Experimentalmodell von Streßgeschwüren bei peroraler Verabreichung

| Verbindung | Anzahl der Tiere | Dosis, mg/kg | Ulkus-index | Prozent der Unterdrückung des Ulkusindexes |
|---|---|---|---|---|
| Kontrolle | 12 | - | 55,1 | - |
| 1) (±)I | 12 | 1 | 4,33 | 92,1 |
| 2) (+)I | 12 | 1 | 7,00 | 87,3 |
| 3) (-)I | 12 | 1 | 39,58 | 28,20 |
| Kontrolle | 52 | - | 71,8 | - |

Wie aus der Tabelle 1 ersichtlich, besitzt die höchste Aktivität die Verbindung (±)I - 92,1 % Unterdrückung des Geschwürindexes. Eine ähnliche Aktivität wird auch bei ihrem rechtsdrehenden Isomer (+)I beobachtet. Das linksdrehende Isomer (-)I besitzt eine vierfach niedrigere Aktivität mit 28,2 % Unterdrückung des Geschwürindexes im Vergleich mit (+)I. Der Austausch des Chloratoms gegen Wasserstoff im Phenyl-Austauschstoff bei 4 führt zur Verminderung der Aktivität.

$LD_{50}$ von (+)I oral in Mäusen beträgt 250 mg/kg. $LD_{50}$ von (+)I intraperitoneal in Mäusen beträgt 125 mg/kg.

Beispiel 2

Bestimmung des Antigeschwüreffektes von (±)I anhand des Experimentalmodells von Wasser-Immersions-Streßgeschwüren bei Ratten.

Die Prüfmethode ist dieselbe wie in Beispiel 1.

Die Ergebnisse der Untersuchungen sind in der Tabelle 3 aufgeführt.

TABELLE 2

Einfluß von (±)I auf ein Modell von experimentellen Wasser-Immersions-Streßgeschwüren bei oraler Anwendung

| Einführungsdosis mg/kg | Anzahl der Tiere | Ulkus-index | Prozent der Unterdrückung des Ulkusindexes |
|---|---|---|---|
| Kontrolle | 20 | 76,6 | - |
| 0,100 | 20 | 41,3 | 46 |
| 0,250 | 20 | 32,7 | 57,3 |
| 0,500 | 20 | 18,8 | 75,5 |
| 1,000 | 20 | 11,3 | 85,00 |

Aus den Ergebnissen der Tabelle 2 ist ersichtlich, daß die Verbindung (±)I, oral angewandt, eine hohe Ulkusaktivität schon bei Dosen von 0,100 mg/kg besitzt. Der Ulkusindex vermindert sich bei dieser Dosis von 76,6 bei den Kontrolltieren auf 41,3, der Unterdrückungsprozentsatz beträgt 46. Die Erhöhung der Dosis führt zur gesetzmäßigen Verstärkung der Ulkusaktivität, wobei ein maximaler Prozentsatz der Unterdrückung des Ulkusindexes bei einer Dosis von 1 mg/kg erreicht wird.

Die Vergleichsuntersuchungen von (±)I und Ranitidin im Streßgeschwürtest zeigen, daß (±)I einen mehrfach höheren Ulkuseffekt gegenüber der Vergleichsverbindung (Tabelle 4) besitzt.

TABELLE 3

Einfluß von Ranitidin auf das Experimentalmodell von Wasser-Immersions-Streßgeschwüren bei Ratten bei peroraler Anwendung

| Einführungsdosis mg/kg | Anzahl der Tiere | Ulkus-index | Prozentsatz der Unterdrückung des Ulkusindexes |
|---|---|---|---|
| Kontrolle | 10 | 44,3 | - |
| 0,5 | 10 | 32,3 | 27 |
| 1 | 10 | 26,9 | 39,27 |
| 3 | 10 | 28,4 | 35,89 |
| 10 | 10 | 22,6 | 48,98 |
| 30 | 10 | 17,0 | 61,62 |

So zum Beispiel ist bei einer Dosis von 0,1 mg/kg von (±)I der Unterdrückungsprozentsatz des Ulkusindexes 46, aber bei fünffach höherer Dosis von Ranitidin - 0,5 mg/kg beträgt der Unterdrückungsprozentsatz kaum 27. Bei gleichen Dosen von (±)I und Ranitidin 1 mg/kg, beträgt der Unterdrückungsprozentsatz für (±)I 85 gegenüber 39,27 für Ranitidin.

Beim Vergleich der Ergebnisse von Tabelle 3 und 4 wird ersichtlich, daß die Verbindung (±)I eine 30-fach höhere Ulkusaktivität im Vergleich zu Ranitidin besitzt, da der maximale Unterdrückungsprozentsatz des Ulkusindexes für Ia bei einer Dosis von 1 mg/kg erreicht wird, während beim Ranitidin eine Dosis von 30 mg/kg zum Erreichen des maximalen Unterdrückungsprozentsatzes notwendig ist.

Beispiel 3

Bestimmung des Ulkuseffektes von (±)I anhand des Experimentalmodells von durch Indometacin hervorgerufenen Geschwüren bei Ratten.

Die Versuche werden mit 50 weißen, männlichen Ratten der Rasse Wistar mit einem Gewicht von 170 bis 200 g durch-geführt. Achtzehn Stunden vor dem Versuch werden die Tiere nicht gefüttert bei freiem Zugang zu Wasserquellen. Das Indometacin wird in einer Dosis von 35 mg/kg intraperitoneal eine halbe Stunde nach der peroralen Verabreichung der Verbindung (±)I, eingeführt. In der fünften Stunde werden die Tiere getötet, die Mägen werden herausgenommen und es werden makroskopisch die destruktiven Veränderungen bestimmt.

TABELLE 4

Einfluß von (±)I auf das Experimentalmodell von durch Indometacin hervorgerufenen Geschwüren bei peroraler Anwendung

| Einführungsdosis mg/kg | Anzahl der Tiere | Ulkus-index | Prozentsatz der Unterdrückung des Ulkusindexes |
|---|---|---|---|
| Kontrolle | 10 | 19,5 | - |
| 0,050 | 10 | 12,2 | 37 |
| 0,250 | 10 | 10,4 | 40,6 |
| 0,500 | 10 | 9,3 | 52 |
| 1,100 | 10 | 4,2 | 78 |

Wie aus der Tabelle 4 ersichtlich ist, besitzt (±)I eine hohe Ulkusaktivität, die schon bei minimaler Dosis von 0,1 mg/kg beobachtet wird. Der Ulkusindex von 19,5 bei den Kontrolltieren sinkt auf 12,2, während der

Unterdrükkungsprozentsatz 37 beträgt. Eine Erhöhung der Dosis führt zu einer gesetzmäßigen Verstärkung des Ulkusindexes, wobei dieser Effekt am stärksten bei der maximal angewandten Dosis von 1 mg/kg ist.

Beispiel 4

Bestimmung des Ulkuseffektes von (±)I anhand des Experimentalmodells von durch Reserpin hervorgerufenen Geschwüren bei Ratten.

Die Versuche werden mit 180 weißen, männlichen Ratten - Rasse Wistar - mit einem Gewicht von 180 bis 270 g durchgeführt. Die Tiere werden 48 Stunden nicht gefüttert bei freiem Zugang zu Wasserquellen. Die Geschwüre wurden hervorgerufen durch die Injektion von 5 mg/kg Reserpin. Eine halbe Stunde vor Einführung des Reserpins erhalten die Tiere peroral verschiedene Dosen von (±)I, die Kontrolltiere Wasser, 24 Stunden nach Einführung des Reserpins werden die Tiere mit Thiopental getötet. Die destruktiven Magenveränderungen werden makroskopisch bestimmt. Es wurden Vergleichsuntersuchungen mit Cimetidin und Ranitidin (Tabelle 5) durchgeführt.

TABELLE 5

Vergleichsuntersuchungen von (±)I mit Cimetidin und Ranitidin auf das Experimentalmodell von durch Reserpin hervorgerufenen Geschwüren

| Verbindung | Einführungsdosis mg/kg | Anzahl der Tiere | Ulkusindex | Prozentsatz der Unterdrückung des Ulkusindexes |
|---|---|---|---|---|
| Kontrolle | - | 16 | 44,58 | - |
| (±)I | 0,250 | 8 | 41,5 | 6,9 |
| (±)I | 0,500 | 12 | 37 | 17 |
| (±)I | 1 | 16 | 23,5 | 47,2 |
| (±)I | 1,5 | 11 | 21,33 | 52,15 |
| (±)I | 2 | 12 | 19,00 | 57,37 |
| (±)I | 3 | 9 | 26,00 | 41,00 |
| Cimetidin | 100 | 8 | 27,62 | 38,00 |
| Ranitidin | 30 | 16 | 23,00 | 48,40 |

Wie aus der Tabelle 5 ersichtlich, liegt der Ulkuseffekt von (±)I nach diesem Test mehrfach höher als der der Vergleichspräparate Cimetidin und Ranitidin, da der maximale Effekt von (±)I bei Dosen von 1,5 und 2 mg/kg erreicht wird, während die benötigten Dosen zum Erreichen des maximalen Effektes von Cimetidin und Ranitidin entsprechend 100 mg/kg und 30 mg/kg betragen.

Beispiel 5

Bestimmung des Ulkuseffektes von (±)I anhand des Experimentalmodells für Geschwüre nach Shei.

Die Versuche wurden mit weißen, männlichen Ratten, Rasse Wistar, mit einem Gewicht von 180 bis 270 g durchgeführt. Die Tiere werden 48 Stunden nicht gefüttert bei freiem Zugang zu Wasserquellen. 5 Stunden nach der Ligatur werden die Tiere getötet, die Mägen werden herausgenommen und die destruktiven Veränderungen makroskopisch untersucht. Als Vergleichspräparate wurden Cimetidin und Ranitidin angewandt (Tabelle 7).

TABELLE 6

Vergleichsuntersuchungen von (±)I mit Cimetidin und Ranitidin anhand des Experimentalmodells von Geschwüren nach Shei

| Verbindung | Einführungsdosis mg/kg | Anzahl der Tiere | Ulkusindex | Prozentsatz der Unterdrückung des Ulkusindexes |
|---|---|---|---|---|
| Kontrolle | - | 21 | 16 | - |
| (±)I | 0,100 | 11 | 22 | + 37,5 |
| (±)I | 0,500 | 11 | 13 | - 18,75 |
| (±)I | 1,000 | 20 | 8,25 | - 48,43 |
| (±)I | 1,5 | 15 | 7,13 | - 55,43 |
| Cimetidin | 100 | 16 | 12,2 | - 23,75 |
| Ranitidin | 30 | 16 | 9 | - 43,75 |

Die Ergebnisse von den Untersuchungen zeigen, daß der Ulkuseffekt von (±)I 30 bis 100-mal höher als der der Vergleichspräparate Cimetidin und Ranitidin liegt, da der maximale Effekt von (±)I bei Dosen von 1 bis 1,5 mg/kg erreicht wird, während die benötigten Dosen zum Erreichen des maximalen Effektes von Cimetidin und Ranitidin entsprechend 100 mg/kg und 30 mg/kg betragen.

Beispiel 6

Herstellung von /-/-2-Methyl-4-/4-chlorphenyl/8-amino-1,2,3,4-tetrahydroisochinolin. (-) II 2,00 g von (-)II und 3,13 g /-/-/2R,3R/-0,0-Di/p-Toluyl-Wein-säure werden in 1000 ml Isopropanol gelöst. Nach Lagerung während einer Nacht bei 4-5° C kristallisieren 0,90 g Di-/p-Toluyl-Tartrat von (-)II (17 %) mit Schmp 189-190° C und $/\alpha/_D^{20}$ = - 53,43/ c = 0,2 Chloroform/. Die zweite Umkristallisierung erfolgt mit 240 ml Isopropanol, wobei 0,51 g Tartrat mit Schmp 193 - 194° C und $/\alpha/_D^{20}$ 3 - 110.24/ c = 0,17, Methanol/ erhalten werden. Durch Behandlung des so erhaltenen Tartrats mit einer wässrigen Ammoniaklösung und anschließender Extraktion mit Chloroform erhält man die freie Base (-)II in Form eines Öles; dieselbe besitzt $/\alpha/_D^{20}$ = - 88,35//c = 0,21, Chloroform/.

Beispiel 7

Herstellung von /+/-2-Methyl-4-/4-chlorphenyl/-8-amino-1,2,3,4-tetrahydroisochinolin. (÷)II

Die Isopropanollösung wird nach Abscheidung der Kristalle des Diastereoisomersalzes von (-)II konzentriert, wobei 0,75 g Salz mit Schmp 186 - 188° C, ausgeschieden werden. Dieses wird filtiert. Das Filtrat wird bis zur Trockenmasse verdampft, wobei ein Rest von 2,35 g verbleibt. Nach Behandlung des letzteren mit Ammoniak und Extraktion mit Chloroform und Verdampfen bis zur Trockenmasse erhält man 0,39 g nicht gereinigte freie Base (=)II in Form von Öl. Diese wird mit einer äquimolekularen Menge von /+/-/ 2s, 3S/-0,0-Di-/p-Toluyl-Weinsäure in Isopropanollösung behandelt. Das erhaltene Tartrat von (+)II wird mit 120 ml Isopropanol umkristallisiert, wobei man 0,66 g gereinigten Tartrats mit Schmp 190 - 192° C und $/\alpha/_D^{20}$ = + 120,79/ c = 0,20 Methanol/ erhält. Die freie Base erhält man nach der im Beispiel 6 beschriebenen Art und Weise und sie besitzt $/\alpha/_D^{20}$ = + 78,85/ c = 0,21, Chloroform/.

Beispiel 8

Herstellung von (+)-2-Methyl-4-(4-chlorphenyl)-8-ethoxycarbonylamino-1,2,3,4-tetrahydroisochinolin (+)I.

2,00 g (0,0073 Mol) von (+)II werden in 18 ml Benzol gelöst und der erhaltenen Lösung wird eine Lösung von 1,06 g (0,098 Mol) von Ethylchlorformiat in 12 ml Benzol zugegeben. Das Reaktionsgemisch siedet 3 Stunden, anschließend wird das Lösungsmittel bei vermindertem Druck entfernt. Der erhaltene Rest wird in 1,8 ml Wasser gelöst. Die Lösung wird mit einer 30 % wässerigen Natronlauge alkalisiert und mit 3 x 20 ml Chloroform extrahiert. Die vereinigten Chloroform-Extrakte werden mit Wasser bis zur neutralen Reaktion gewaschen und mit Natriumsulfat getrocknet. Nach Abtrennung des Chloroforms unter Vakuum erhält man 2,54 g (97 %) (+)I freie Base. Letzeres wird in ein saures Maleat durch heißes Lösen in 6 ml Aceton umgewandelt und der erhaltenen Lösung wird eine heiße Lösung von 0,95 g (0,008 Mol) Maleinsäure in 3 ml Aceton zugegeben. Es wird mit 40 ml Aceton verdünnt und mit Hexan koaguliert, wobei man 3,47 g (99 %) von (+)I saurem Maleat mit Schmp. 145-148°C und $/\alpha/_D^{20}$ = + 41,50 (c = 0,59, Methanol) erhält.

Auf analoge Art und Weise erhält man:

(-)-2-Methyl-4-/4-chlorphenyl)-8-ethyloxycarbonylamino-1,2,3,4-tetrahydroisochinolin-saures Maleat (-)I mit Schmp. 145-147°C und $/\alpha/_D^{20}$ = -40,32 (c = 0,53 Methanol), erhalten vom Ausgangs (-)II.

## Ansprüche

1. Verwendung von 2-Methyl-4-(4-chlorphenyl)-8-ethoxycarbonylamino 1,2,3,4-tetrahydroisochinolin (±)I, seines rechtsdrehenden Isomers (+)I und/oder seiner optisch aktiven Isomeren zur Herstellung von Ulkusmitteln.

## Claims

1. Use of 2-methyl-4-(4-chlorophenyl)-8-ethoxycarbonylamino-1,2,3,4-tetrahydroisoquinoline (±)I, its dextrorotary isomer (+)I and/or its optically active isomers for the production of ulcer agents.

## Revendications

1. Utilisation de la méthyl-2 (chloro-4 phényl)-4 éthorycarbonyl-8 tétrahydro-1,2,3,4 isoquinoléine (±)I, de son isomère dextrogyre (+)I et/ou de ses isomères optiquement actifs pour la fabrication d'agents anti-ulcère.